(19) Europäisches Patentamt
European Patent Office
Office européen des brevets

(11) Veröffentlichungsnummer: **0 272 621 B1**

(12) **EUROPÄISCHE PATENTSCHRIFT**

(45) Veröffentlichungstag der Patentschrift: **27.11.91**

(51) Int. Cl.5: **C07C 49/675**, C07C 45/46

(21) Anmeldenummer: **87118716.7**

(22) Anmeldetag: **17.12.87**

# (54) Verfahren zur Herstellung von 5H-Dibenzo[a,d]cyclohepten-5-onen.

(30) Priorität: **24.12.86 DE 3644462**

(43) Veröffentlichungstag der Anmeldung:
**29.06.88 Patentblatt 88/26**

(45) Bekanntmachung des Hinweises auf die Patenterteilung:
**27.11.91 Patentblatt 91/48**

(84) Benannte Vertragsstaaten:
**AT BE CH DE ES FR GB GR IT LI NL SE**

(56) Entgegenhaltungen:
**CH-A- 503 686**

**JOURNAL OF MEDICINAL CHEMISTRY, Band 20, Nr. 12, 1977, S. 1557-1562; J. P. DUNN et al, "Dibenzotroponeacetic and -propionic acids. Potent new antiinflammatory agents"**

**CHEMISCHE BERICHTE, Band 83, Nr. 3, 1950, S. 367-371; W. TREIBS et al, "Ueber das 1.2;4.5-Dibenzosuberon-(3)"**

**CHEMISCHE BERICHTE, Band 84, Nr. 8, 1951, S. 671-679; W. TREIBS et al, "Synthese des 2,3;6.7-Debenzo-suberen-(4)-ons(1) und des 2,3;6.7-Dibenz-suberens-(4)"**

**THE JOURNAL OF THE AMERICAN CHEMICAL SOCIETY, Band 73, 1951, S. 1673-1678; A. C. COPE et al, "Cyclic polyolefins. XV. 1-Methylene-2,3,6,7-dibenzcycloheptatriene"**

(73) Patentinhaber: **BASF Aktiengesellschaft**
**Carl-Bosch-Strasse 38**
**W-6700 Ludwigshafen(DE)**

(72) Erfinder: **Thyes, Marco, Dr.**
**Thorwaldsenstrasse 1**
**W-6700 Ludwigshafen(DE)**
Erfinder: **Steiner, Gerd, Dr.**
**Oberer Waldweg 1**
**W-6719 Kirchheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft ein neues Verfahren zur Herstellung von 5H-Dibenzo[a,d]cyclohepten-5-onen der allgemeinen Formel I,

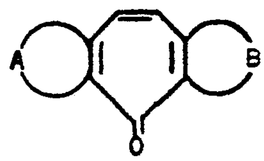

(I)

in der die Reste A und B Reste zur Vervollständigung von aromatischen Ringsystemen bedeuten.

Aus J. Med. Chem. 20, (1977) 1557 ist es bekannt, daß die direkte Cyclisierung der (E/Z)-2-(2-m-Tolylethenyl)-benzoesäure (im Verhältnis E:Z = 1:1) nicht zu einem tricyclischen Keton des Typs I, sondern zu einem 6-Ringlacton

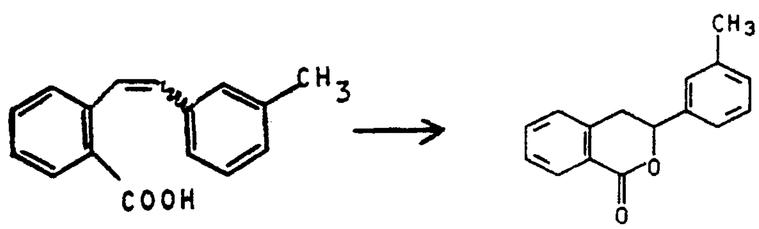

führt.

Ferner ist es bekannt, daß die Verbindungen des Typs I durch Hydrierung der Ethenyldoppelbindung in E/Z-Verbindungen II′,

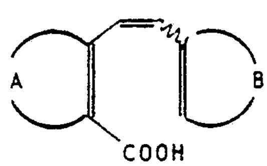

(II′)

Cyclisierung und anschließender Wiedereinführung der zuvor entfernten Doppelbindung herstellbar sind [z.B. J. Med. Chem. 20, 1557 (1977); J. Med. Chem. 22, 1357 (1979); Chem. Ber. 84, 671 (1951) oder J. Am. Chem. Soc. 73, 1673 (1951)].

Der Erfindung lag die Aufgabe zugrunde, ein Verfahren zu entwickeln, um auf kürzerem Wege zu den 5H-Dibenzo[a,d]cyclohepten-5-onen I zu gelangen.

Demgemäß wurde ein Verfahren zur Herstellung von 5H-Dibenzo[a,d]cyclohepten-5-onen der allgemeinen Formel I

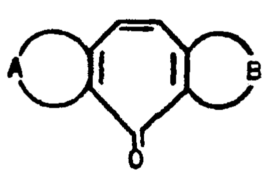

(I)

in der die Reste A und B Reste zur Vervollständigung von aromatischen Ringsystemen bedeuten, gefunden, welches dadurch gekennzeichnet ist, daß man (Z)-2-(2-Arylethenyl)-arylcarbonsäuren der allgemeinen Formel II

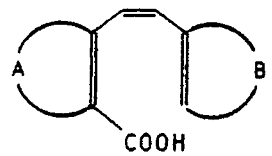

(II)

mit einem Carbonsäure-Halogenierungsmittel bei Temperaturen von (-30) bis +120 °C in die entsprechenden Carbonsäurehalogenide der allgemeinen Formel III,

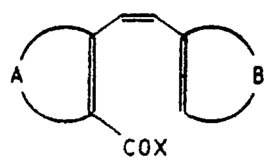

(III)

in der der Rest X für Halogen steht, überführt und diese bei 0 bis 100 °C in Gegenwart einer Lewis-Säure cyclisiert.

Die A und B enthaltenden Ringe entsprechen dem Benzol und annelierten Benzolen wie 1,2- und 2,3-Naphthalin und deren bis zu dreifach substituierten Derivaten.

Als Substituenten kommen in Betracht:

- Halogen, wie Fluor, Chlor oder Brom, besonders Fluor und Chlor,
- Trifluormethyl
- $C_1$-$C_8$-Alkyl, besonders $C_1$-$C_4$-Alkyl wie Methyl, Ethyl, n-Propyl, iso-Propyl, n-Butyl, iso-Butyl, sec.-Butyl, tert.-Butyl,
- $C_1$-$C_8$-Alkoxy, besonders $C_1$-$C_4$-Alkoxy wie Methoxy, Ethoxy, n-Propoxy, Isopropoxy, n-Butoxy, iso-Butoxy, sec.-Butoxy, tert.-Butoxy.

Vorzugsweise befinden sich die Substituenten an dem die Gruppierung B enthaltenden Ring.

Als Beispiele für bevorzugte Ausgangsverbindungen II seien diejenigen genannt, welche den folgenden bevorzugten Verfahrensprodukten entsprechen:

5H-Dibenzo[a,d]cyclohepten-5-on;
1-Chlor-5H-dibenzo[a,d]cyclohepten-5-on;
2-Chlor-5H-dibenzo[a,d]cyclohepten-5-on;
3-Chlor-5H-dibenzo[a,d]cyclohepten-5-on;
1-Brom-5H-dibenzo[a,d]cyclohepten-5-on;
2-Brom-5H-dibenzo[a,d]cyclohepten-5-on;
3-Brom-5H-dibenzo[a,d]cyclohepten-5-on;
1-Fluor-5H-dibenzo[a,d]cyclohepten-5-on;
2-Fluor-5H-dibenzo[a,d]cyclohepten-5-on;
3-Fluor-5H-dibenzo[a,d]cyclohepten-5-on;
1,2-Dichlor-5H-dibenzo[a,d]cyclohepten-5-on;
1,4-Dichlor-5H-dibenzo[a,d]cyclohepten-5-on;
2,3-Dichlor-5H-dibenzo[a,d]cyclohepten-5-on;
2,4-Dichlor-5H-dibenzo[a,d]cyclohepten-5-on;
1,2-Dibrom-5H-dibenzo[a,d]cyclohepten-5-on;
2,3-Dibrom-5H-dibenzo[a,d]cyclohepten-5-on;
2-Trifluormethyl-5H-dibenzo[a,d]cyclohepten-5-on;
1-Methyl-5H-dibenzo[a,d]cyclohepten-5-on;
2-Methyl-5H-dibenzo[a,d]cyclohepten-5-on;
3-Methyl-5H-dibenzo[a,d]cyclohepten-5-on;
1-Ethyl-5H-dibenzo[a,d]cyclohepten-5-on;
2-Ethyl-5H-dibenzo[a,d]cyclohepten-5-on;
3-Ethyl-5H-dibenzo[a,d]cyclohepten-5-on;
2-Propyl-5H-dibenzo[a,d]cyclohepten-5-on;
2-Isopropyl-5H-dibenzo[a,d]cyclohepten-5-on;
2-Butyl-5H-dibenzo[a,d]cyclohepten-5-on;

1,2-Dimethyl-5H-dibenzo[a,d]cyclohepten-5-on;
2,3-Dimethyl-5H-dibenzo[a,d]cyclohepten-5-on;
2,4-Dimethyl-5H-dibenzo[a,d]cyclohepten-5-on;
1-Methoxy-5H-dibenzo[a,d]cyclohepten-5-on;
2-Methoxy-5H-dibenzo[a,d]cyclohepten-5-on;
3-Methoxy-5H-dibenzo[a,d]cyclohepten-5-on;
1-Ethoxy-5H-dibenzo[a,d]cyclohepten-5-on;
2-Ethoxy-5H-dibenzo[a,d]cyclohepten-5-on;
3-Ethoxy-5H-dibenzo[a,d]cyclohepten-5-on;
1,2-Dimethoxy-5H-dibenzo[a,d]cyclohepten-5-on;
2,3-Dimethoxy-5H-dibenzo[a,d]cyclohepten-5-on;
1,4-Dimethoxy-5H-dibenzo[a,d]cyclohepten-5-on;

Die (Z)-2-(2-Arylethenyl)-arylcarbonsäuren II sind z.T. bekannt (z.B. J. Org. Chem. 43, (1978) 3283) oder können nach bekannten Methoden durch Wittig-Reaktion hergestellt werden. Die Isolierung der Z-Isomeren aus E/Z-Gemischen ist gegebenenfalls durch Kristallisation oder Chromatographie möglich.

Es eignen sich auch E/Z-Isomerengemische, besonders solche mit einem hohen Z-Isomerenanteil. Beim Vorliegen eines E/Z-Isomerengemisches kann es vorteilhaft sein, das E-Isomere über die Reaktionsstufen mitzuschleppen, denn dies führt in der Regel zu leichter abtrennbaren Endprodukten und wirkt sich auch nicht hinderlich auf die Reaktion des Z-Isomeren aus.

Im folgenden wird das erfindungsgemäße Verfahren anhand des Z-Isomeren beschrieben. Es ist genauso für das E/Z-Gemisch anwendbar.

Herstellung der Carbonsäurehalogenide III

In einem inerten Lösungsmittel werden eine (Z)-2-(2-Arylethenyl)-arylcarbonsäure II und gegebenenfalls ein säurebindendes Mittel vorgelegt und bei Temperaturen von (-30) bis +120 °C, vorzugsweise 20 bis 80 °C, durch Zutropfen eines Carbonsäure-Halogenierungsmittels umgesetzt.

Als säurebindende Mittel eignen sich beispielsweise organische Basen wie Triethylamin oder Pyridin. Bei deren Anwesenheit ist es zweckmäßig, das Produkt III zu isolieren. Bei der direkten Weiterverarbeitung der rohen Carbonsäurehalogenide III arbeitet man vorzugsweise ohne säurebindendes Mittel.

Als Carbonsäure-Halogenierungsmittel sind Thionylchlorid, Thionylbromid, Phosphoroxychlorid, Phosphortrichlorid, Phosphorpentachlorid, Phosphortribromid und Phosphorpentabromid bevorzugt. Besonders bevorzugt wird Thionylchlorid.

Bevorzugt wird mit äquimolaren Mengen oder einem Überschuß, vorzugsweise mit bis zu 3 Äquivalenten von II, des Carbonsäure-Halogenierungsmittels gearbeitet. Besonders bevorzugt wird mit äquimolaren Mengen oder einem bis zu 5-mol-%igen Überschuß des Carbonsäure-Halogenierungsmittels gearbeitet. Dies empfiehlt sich vor allem bei der direkten Weiterverarbeitung der rohen Carbonsäurehalogenide III.

Als Lösungsmittel eignen sich beispielsweise halogenierte Kohlenwasserstoffe wie Methylenchlorid, Chloroform, Ethylenchlorid, 1,1,1-Trichlorethan, 1,1,2,2-Tetrachlorethan, Chlorbenzol oder Brombenzol oder aromatische Kohlenwasserstoffe wie Benzol, Toluol oder die Xylole. Bevorzugt wird Methylenchlorid, vor allem bei der direkten Weiterverarbeitung.

Herstellung der 5H-Dibenzo[a,d]cyclohepten-5-one I

Das Carbonsäurehalogenid III wird vorzugsweise in einem für die Friedel-Crafts-Acylierung inerten Lösungsmittel, z.B. Chlorkohlenwasserstoffen, wie Methylenchlorid, Ethylenchlorid, 1,1,1-Trichlorethan oder 1,1,2,2-Tetrachlorethan bevorzugt in Methylenchlorid vorgelegt und bei Temperaturen von 0 bis 100 °C, vorzugsweise 10 bis 80 °C, besonders bevorzugt bei 20 bis 30 °C, durch portionsweise Zugabe einer festen Lewis-Säure oder durch Zutropfen einer Suspension oder Aufschlämmung einer Lewis-Säure in einem der inerten Lösungsmittel umgesetzt.

Als Lewis-Säuren eignen sich die üblichen Säuren, bevorzugt wird Aluminiumtrichlorid.

Bevorzugt wird mit äquimolaren Mengen oder einem Überschuß, vorzugsweise 1,2 bis 1,5 mol Lewis-Säure pro Mol III, gearbeitet.

In der bevorzugten Isolierungsmethode des 5H-Dibenzo[a,d]cyclohepten-5-ons I wird zu dem Hydrolysegemisch zwecks Verringerung der Löslichkeit des Dibenzocycloheptenons in der organischen Phase ein unpolares, organisches Lösungsmittel, beispielsweise Petrolether mit einem Siedebereich von 40 bis 60 °C oder Cyclohexan, gegeben und das Dibenzocycloheptenon dann mittels Filtration abgetrennt. Die Menge des unpolaren, organischen Lösungsmittels kann dabei beispielsweise so gewählt sein, daß das Volumenverhältnis des für die Umsetzung verwendeten Lösungsmittels und dieses unpolaren, organischen Lösungsmittels zwischen etwa 0,2:1 und 0,6:1 liegt.

Die Verbindungen I dienen als Zwischenprodukte für die Herstellung pharmakologisch aktiver 5-Cyanmethylen-5H-dibenzo[a,d]cycloheptene (DE-OS 30 09 034). Das (Z)-[2-Chlor-10-(4-methyl-1-piperazinyl)-5H-dibenzo[a,d]cyclohepten-5-yliden]acetonitril, Smp. 229 bis 231 °C, konnte über die Zwischenstufen 2-Chlor-10,11-dibrom-10,11-dihydro-5H-dibenzo[a,d]cyclohepten-5-on (hergestellt durch Addition von Brom an die olefinische Doppelbindung des 2-Chlor-5H-dibenzo[a,d]cyclohepten-5-ons),Smp. 161-164 °C, das Gemisch aus 10-Brom-2-chlor-5H-dibenzo[a,d]cyclohepten-5-on und 11-Brom-2-chlor-5H-dibenzo[a,d]-cyclohepten-5-on im Verhältnis von 15:85, Smp. 134-137 °C, und ein Gemisch aus (E/Z)-[10-Brom-2-chlor-5H-dibenzo[a,d]cyclohepten-5-yliden]acetonitril und (E/Z)-[11-Brom-2-chlor-5H-dibenzo[a,d]cyclohepten-5-yliden]acetonitril, Smp. 97 bis 100 °C, erhalten werden. Ferner konnte das pharmakologisch aktive Gemisch aus (E/Z)-[2-Fluor-10-(4-methyl-1-piperazinyl)-5H-dibenzo[a,d]cyclohepten-5-yliden]acetonitril (Hemihydrate) und (E/Z)-[2-Fluor-11-(4-methyl-1-piperazinyl)-5H-dibenzo[a,d]cyclohepten-5-yliden]acetonitril (Hemihydrate), Smp. 98 bis 101 °C, über 2-Fluor-5H-dibenzo[a,d]cylohepten-5-on, das pharmakologisch aktive Gemisch aus (E/Z)-[1-Chlor-10-(4-methyl-1-piperazinyl)-5H-dibenzo[a,d]cyclohepten-5-yliden]-acetonitril und (E/Z)-[1-Chlor-11-(4-methyl-1-piperazinyl)-5H-dibenzo[a,d]cyclohepten-5-yliden]acetonitril, Smp. 101-104 °C, über 1-Chlor-5H-dibenzo[a,d]cyclohepten-5-on sowie das pharmakologisch aktive Gemisch aus (E/Z)-[3-Chlor-10-(4-methyl-1-piperazinyl)-5H-dibenzo[a,d]cyclohepten-5-yliden]acetonitril und (E/Z)-[3-Chlor-11-(4-methyl-1-piperazinyl)-5H-dibenzo[a,d]cyclohepten-5-yliden]acetonitril, Smp. 102-105 °C, über 3-Chlor-5H-dibenzo[a,d]cyclohepten-5-on erhalten werden.

Beispiel 1

2-Chlor-5H-dibenzo[a,d]cyclohepten-5-on

Zu 500 g (1,933 mol) (E/Z)-2-[2-(3-Chlorphenyl)ethenyl]benzoesäure (im Verhältnis von etwa 23 : 77) in 500 ml Methylenchlorid wurden bei Rückflußtemperatur unter Rühren innerhalb von 1 Stunde 241,6 g (2,031 mol) Thionylchlorid getropft und 1 Stunde gerührt. Anschließend wurde bei Raumtemperatur innerhalb von 45 Min. eine Suspension von 333 g (2,497 mol) wasserfreiem Aluminiumchlorid in 100 ml Methylenchlorid zugegeben, 30 Min. bei Raumtemperatur gerührt und das Reaktionsgemisch dann unter Rühren auf ein Gemisch aus 3 kg Eis und 1 l Wasser gegossen. Das Hydrolysegemisch wurde ca. 30 Min. gerührt und dann unter Rühren mit 1 l Petrolether (Siedebereich 40 bis 60 °C) versetzt. Es wurde nochmals ca. 30 Min. gerührt und danach abgesaugt. Der Filterrückstand wurde zuerst mit 5 l Wasser, dann noch mit 1 l Petrolether (Siedebereich 40 bis 60 °C) gewaschen und getrocknet. Isoliert wurden 270 g 2-Chlor-5H-dibenzo[a,d]cyclohepten-5-on in 58%iger Ausbeute; Schmelzpunkt 160-161 °C.

Beispiel 2

2-Fluor-5H-dibenzo[a,d]cyclohepten-5-on

Zu 18,2 g (75,1 mmol) (E/Z)-2-[2-(3-Fluorphenyl)ethenyl]benzoesäure (im Verhältnis von etwa 27 : 73) in 50 ml Methylenchlorid wurden bei Rückflußtemperatur unter Rühren 9,4 g (79 mmol) Thionylchlorid innerhalb von 20 Min. getropft und 1 Stunde nachgerührt. Anschließend wurden bei Raumtemperatur innerhalb von 10 Min. 12,9 g (97 mmol) wasserfreies Aluminiumchlorid zudosiert, 30 Min. bei 20-25 °C gerührt und wie üblich aufgearbeitet. Isoliert wurden 8,4 g 2-Fluor-5H-dibenzo[a,d]cyclohepten-5-on in 50%iger Ausbeute; Schmelzpunkt 119-123 °C.

**Patentansprüche**

**1.** Verfahren zur Herstellung von 5H-Dibenzo[a,d]cyclohepten-5-onen der allgemeinen Formel I,

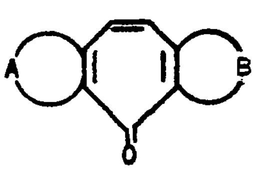

(I)

in der die Reste A und B Reste zur Vervollständigung aromatischer Ringsysteme bedeuten, dadurch gekennzeichnet, daß man (Z)-2-(2-Arylethenyl)-arylcarbonsäuren der allgemeinen Formel II

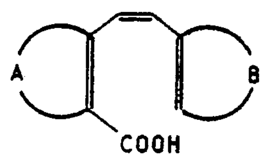

(II)

mit einem Carbonsäure-Halogenierungsmittel bei (-30) bis +120 °C in die entsprechenden Carbonsäurehalogenide der allgemeinen Formel III,

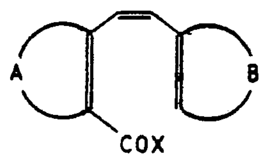

(III)

in der der Rest X für Halogen steht, überführt und diese bei 0 bis 100 °C in Gegenwart einer Lewis-Säure cyclisiert.

2. Verfahren nach Anspruch 1, dadurch gekennzeichnet, daß man ein (E/Z)-Isomerengemisch der 2-(2-Arylethenyl)-arylcarbonsäuren II verwendet.

3. Verfahren nach den Ansprüchen 1 und 2, dadurch gekennzeichnet, daß man als Halogenierungsmittel Thionylchlorid, Phosphoroxychlorid, Phosphortrichlorid, Phosphorpentachlorid, Phosphortribromid oder Phosphorpentabromid verwendet.

4. Verfahren nach den Ansprüchen 1 bis 3, dadurch gekennzeichnet, daß man die Halogenierung bei 20 bis 80 °C vornimmt.

5. Verfahren nach den Ansprüchen 1 bis 4, dadurch gekennzeichnet, daß man für die Cyclisierung als Lewis-Säure Aluminiumchlorid verwendet.

6. Verfahren nach den Ansprüchen 1 bis 5, dadurch gekennzeichnet, daß man die Cyclisierung bei 20 bis 30 °C vornimmt.

## Claims

1. A process for preparing 5H-dibenzo[a,d]cyclohepten-5-ones of the general formula I

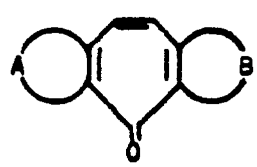

(I)

where radicals A and B are each radicals for completing an aromatic ring system, which comprises converting a (Z)-2-(2-arylethenyl)arylcarboxylic acid of the general formula II

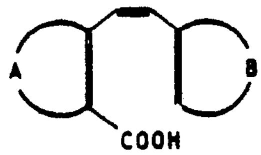

(II)

at from (-30) to +120 °C with a halogenating agent for carboxylic acids into the corresponding carbonyl halide of the general formula III

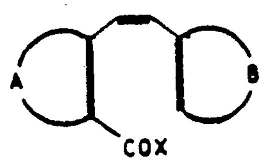

(III)

where X is halogen, and cyclizing the said carbonyl halide at from 0 to 100 °C in the presence of a Lewis acid.

**2.** A process as claimed in claim 1, wherein an (E/Z )-isomer mixture of a 2-(2-arylethenyl)arylcarboxylic acid II is used.

**3.** A process as claimed in claim 1 or 2, wherein the halogenating agent used is thionyl chloride, phosphorus oxychloride, phosphorus trichloride, phosphorus pentachloride, phosphorus tribromide or phosphorus pentabromide.

**4.** A process as claimed in any of claims 1 to 3, wherein the halogenation is carried out at from 20 to 80 °C.

**5.** A process as claimed in any of claims 1 to 4, wherein the Lewis acid for the cyclization is aluminum chloride.

**6.** A process as claimed in any of claims 1 to 5, wherein the cyclization is carried out at from 20 to 30 °C.

**Revendications**

**1.** Procédé de préparation de 5H-dibenzo [a,d] cycloheptène-5-ones de formule générale I

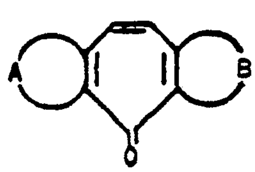

(I)

dans laquelle A et B représentent des radicaux complétant des systèmes cycliques aromatiques, caractérisé en ce que l'on convertit des acides (Z)-2-(2-aryléthényl)-arylcarboxyliques de formule générale II

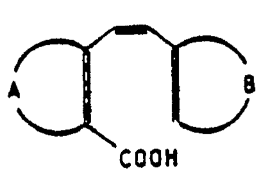

(II)

à l'aide d'un agent halogénant des acides carboxyliques, à des températures de - 30 à + 120 degrés C, en les halogénures d'acides carboxyliques correspondants de formule générale III

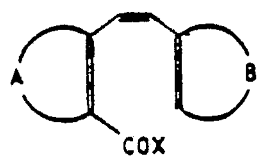

(III)

dans laquelle X représente un halogène, et qu'on cyclise en présence d'un acide de Lewis à des températures de 0 à 100 degrés C.

2. Procédé selon la revendication 1, caractérisé en ce que l'on utilise un mélange des isomères E/Z des acides 2-(2-aryléthényl)-arylcarboxyliques II.

3. Procédé selon les revendications 1 et 2, caractérisé en ce que l'on utilise, en tant qu'agent halogénant, le chlorure de thionyle, l'oxychlorure de phosphore, le trichlorure de phosphore, le pentachlorure de phosphore, le tribromure de phosphore ou le pentabromure de phosphore.

4. Procédé selon les revendications 1 à 3, caractérisé en ce que l'on effectue l'halogénation à des températures de 20 à 80 degrés C.

5. Procédé selon les revendications 1 à 4, caractérisé en ce que l'acide de Lewis utilisé pour la cyclisation est le chlorure d'aluminium.

6. Procédé selon les revendications 1 à 5, caractérisé en ce que l'on effectue la cyclisation à des températures de 20 à 30 degrés C.